# EUROPEAN PATENT SPECIFICATION

(11) **EP 4 313 282 B1**
(45) Date of publication and mention of the grant of the patent: **01.07.2026**
(21) Application number: 22715650.2
(22) Date of filing: 30.03.2022
(51) Int. Cl.: A61N 5/06, A61F 9/008

(54) **GUIDING ADJUSTMENTS OF A LASER SPOT SIZE DURING OPHTHALMIC LASER THERAPY TREATMENT**
FÜHRUNG VON ANPASSUNGEN EINER LASERPUNKTGRÖSSE WÄHREND EINER AUGENLASERTHERAPIEBEHANDLUNG
GUIDAGE DE RÉGLAGES DE LA TAILLE D'UN POINT LASER PENDANT UN TRAITEMENT THÉRAPEUTIQUE PAR LASER OPHTALMIQUE

(30) Priority: 30.03.2021 US 202117216826
(43) Date of publication of application: 07.02.2024
(73) Proprietor: Modulight Corporation, 33720 Tampere (FI)
(72) Inventor: TANILA, Timo, 33480 Ylöjärvi (FI)
(74) Representative: Papula Oy
(86) International application number: PCT/FI2022/050206
(87) International publication number: WO 2022/207980

(56) References cited:
- WO-A2-01/87181
- US-A1- 2019 019 514
- US-B1- 6 312 422

## Description

### TECHNICAL FIELD

Various example embodiments generally relate to the field of medical devices. In particular, some example embodiments relate to an ophthalmic medical device, such as a photodynamic therapy treatment device, and assisting use of a certain size of laser spot for treatment of specific parts of a human eye, and more particularly in guiding correction of the laser spot size by sound.

### BACKGROUND

Ophthalmic medical lasers are typically configured to deliver a certain size laser spot on parts of a human eye for a light-based treatment effect, like photothermal or photodynamic therapy. For example, photodynamic therapy (PDT) devices may be used for operations wherein a photosensitizer or a photosensitizing agent and a particular type of light are used for treatment of a patient. When photosensitizers are exposed to a specific wavelength of light, they produce a form of oxygen that kills nearby cells. In general, an ophthalmic device may be a laser device configured to produce the particular type of light having a specific wavelength.

PDT may be used, for example, to treat vascular issues in the retina and choroid of an eye. During treatment of the eye, it can be inconvenient to start changing settings of the device. Further, improper use of the PDT device may result in incomplete treatment, for example, due to partial photoactivation, overtreat-ment due to over-activation, or damage to surrounding normal tissue. It would be beneficial to improve usability of the PDT and other ophthalmic laser devices.

US 6 312 422 B1 relates to a process and arrangement for monitoring and controlling treatment parameters in an ophthalmic treatment device.

### SUMMARY

This summary is provided to introduce a selection of concepts in a simplified form that are further described below in the detailed description. This summary is not intended to identify key features or essential features of the claimed subject matter, nor is it intended to be used to limit the scope of the claimed subject matter.

According to a first aspect, a device is provided. The device may comprise at least one processor and at least one memory, the memory comprising program code configured to, when executed by the processor, cause the device at least to monitor a current laser spot size of an ophthalmic laser therapy treatment device based on sensor data; cause output of a first sound signal in response to detecting the current laser spot size differs from a required laser spot size; determine a required parameter change to set the value of the current laser spot size to the required laser spot size; determine characteristics of a second sound signal based on the required parameter change, wherein the second sound signal comprises a non-speech sound; and cause output of the second sound signal, wherein characteristics of the second sound signal are indicative of a magnitude of the required parameter change and whether the laser spot size needs to be increased or decreased.

According to an embodiment, the current laser spot size is monitored based on sensor data received from the ophthalmic laser therapy treatment device.

According to an embodiment, in addition or alternatively, the characteristics of the second sound signal comprise a higher or a lower frequency depending on whether the laser spot size needs to be increased or decreased.

According to an embodiment, in addition or alternatively, the characteristics of the second sound signal comprise a different repetition interval based on the magnitude of the required parameter change.

According to an embodiment, in addition or alternatively, the characteristics of the second sound signal comprise a different volume level based on the magnitude of the required parameter change.

According to an embodiment, in addition or alternatively, the characteristics of the second sound signal comprise setting at least two of a different frequency, a different repetition interval or a different volume level based on different magnitudes of the required parameter changes and whether the laser spot size needs to be increased or decreased.

According to an embodiment, in addition or alternatively, the at least one memory and the program code are further configured to, when executed by the processor, cause the device to detect when the current laser spot size corresponds to the predetermined laser spot size; and cause output of a third sound signal indicative of a successful adjustment.

According to an embodiment, in addition or alternatively, at least one of the first sound signal or the third sound signal comprises at least one of a speech or a non-speech sound.

According to an embodiment, in addition or alternatively, the at least one memory and the program code are further configured to, when executed by the processor, cause the device to detect the current laser spot size is changed in response to an adjustment of the laser spot size made by a user; determine a new required parameter change; and change the characteristics of the second sound signal based on the new required parameter change.

According to an embodiment, in addition or alternatively, monitoring the current laser spot size comprises obtaining a value of the current laser spot size based on a position of a laser spot size adjustment wheel of the ophthalmic laser therapy treatment device and comparing the value to the predetermined laser spot size value.

According to a second aspect, a method is provided. The method may comprise monitoring a current laser spot size of an ophthalmic laser therapy treatment device; causing output of a first sound signal in response to detecting the current laser spot size differs from a predetermined laser spot size based on sensor data; determining a required parameter change to set the value of the current laser spot size to the predetermined laser spot size; determining characteristics of a second sound signal based on the required parameter change, wherein the second sound signal comprises a non-speech sound; and causing output of the second sound signal, wherein characteristics of the second sound signal are indicative of a magnitude of the required parameter change and whether the laser spot size needs to be increased or decreased.

According to an embodiment, determining the characteristics of the second sound signal comprises a higher or a lower frequency depending on whether the laser spot size needs to be increased or decreased.

According to an embodiment, in addition or alternatively, determining the characteristics of the second sound signal comprises a different repetition interval based on the magnitude of the required parameter change.

According to an embodiment, in addition or alternatively, determining the characteristics of the second sound signal comprises a different volume level based on the magnitude of the required parameter change.

According to an embodiment, in addition or alternatively, determining the characteristics of the second sound signal comprises setting at least two of a different frequency, a different repetition interval or a different volume level based on different magnitudes of the required parameter changes and whether the laser spot size needs to be increased or decreased.

According to an embodiment, in addition or alternatively, the characteristics of the second sound signal comprise setting at least two of a different frequency, a different repetition interval or a different volume level based on different magnitudes of the required parameter changes and whether the laser spot size needs to be increased or decreased.

According to an embodiment, in addition or alternatively, the method further comprises detecting when the current laser spot size corresponds to the predetermined laser spot size; and causing output of a third sound signal indicative of a successful adjustment.

According to an embodiment, in addition or alternatively, the method comprises detecting the current laser spot size is changed in response to an adjustment of the laser spot size made by a user; determining a new required parameter change; and changing the characteristics of the second sound signal based on the new required parameter change.

According to an embodiment, in addition or alternatively, at least one of the first sound signal or the third sound signal comprises at least one of a speech or a non-speech sound.

According to an embodiment, in addition or alternatively, monitoring the current laser spot size comprises obtaining a value of the current laser spot size based on a position of a laser spot size adjustment wheel of the ophthalmic laser therapy treatment device and comparing the value to the predetermined laser spot size value.

According to a third aspect, a system is provided. The system may comprise the device according to the first aspect communicatively coupled to an ophthalmic laser therapy device.

According to a fourth aspect, a computer program is configured, when executed by a computing device, to cause the device to perform the method according to the second aspect.

Many of the attendant features will be more readily appreciated as they become better understood by reference to the following detailed description considered in connection with the accompanying drawings.

### BRIEF DESCRIPTION OF THE DRAWINGS

The accompanying drawings, which are included to provide a further understanding of the example embodiments and constitute a part of this specification, illustrate example embodiments and together with the description help to explain the example embodiments. In the drawings:
**FIG. 1** illustrates an example of an ophthalmic laser therapy treatment system, according to an example embodiment;
**FIG. 2** illustrates an example embodiment of a device configured to practice one or more example embodiments;
**FIG. 3** illustrates an example of a method for providing audible guidance to a user of an ophthalmic laser therapy treatment device, according to an example embodiment;
**FIG. 4** illustrates an example of a method for determining sound instructions provided for a user of an ophthalmic laser treatment device, according to an example embodiment.

### DETAILED DESCRIPTION

Reference will now be made in detail to example embodiments, examples of which are illustrated in the accompanying drawings. The detailed description provided below in connection with the appended drawings is intended as a description of the present examples and is not intended to represent the only forms in which the present example may be constructed or utilized. The description sets forth the functions of the example and the sequence of operations for constructing and operating the example. However, the same or equivalent functions and sequences may be accomplished by different examples.

An embodiment may be configured to improve usability of a device designed for ophthalmic or specifically PDT treatment. A device and a method are provided accordingly, which may help a user of the ophthalmic laser device to set back a required laser spot size. The device may be configured to provide guidance for the user by a non-speech sound. Hence, the laser spot size may be adjusted by the user during active treatment without requiring the user to have visual instructions.

During ophthalmic treatment for an eye of a patient, a user of the ophthalmic device, e.g. the PDT device, may be required to see a graphical user interface to monitor parameters and progress of the treatment. The graphical user interface may be located inconveniently, and it may be difficult to see by the user. The user of the ophthalmic device may accidentally alter the set laser spot size during treatment and the spot size needs to be readjusted before continuing the treatment. During eye treatment, the user may be holding a contact lens on the eye. If the user moves to glance at the graphical user interface, the contact lens can move or may need to be taken away and positioned again.

Alternatively, data on the graphical user interface may be communicated out loud to the user by another person. However, this requires the presence of the other person during the treatment just for this simple task. Further, when adjustment of the laser spot size is required, the user may need to see a parameter scale of a mechanism configured for adjustment of the laser spot size. In addition, the user may need to check a display showing the required laser spot size to set back the correct value.

According to an example embodiment, a device may be configured to cause a plurality of sound signals to be output based on a value of the laser spot size. The sound signals are configured to guide the user to operate a device configured to generate the laser spot size. The device may be configured to determine one or more characteristics of the sound signals based on a required laser spot size for a treatment session and current value of the laser spot size. The sound signals may be dispatched by the device to another device for outputting audible instructions based on the sound signals. Alternatively, the device may comprise an audio unit configured to output the audible instructions.

The device may select features of the sound signals such that the laser spot size can be corrected by the user in a quick manner just by listening. The sound signals may comprise a warning of an incorrect laser spot size. The sound signals may further comprise a confirmation of a correct laser spot size. The sound signals may comprise indications of how much and towards which direction the laser spot size requires adjustment on the control scale.

Hence, audible instructions may be provided for the user based on the characteristics of the outputted sounds which change based on a difference between the required and current laser spot size value. The provided sound signal guidance enables the user to not need to look at a graphical user interface while adjusting the required laser spot size. Further, the information required for the adjustment may be given more precisely, timely, and in a quicker manner compared to guidance given by another person or the graphical user interface. In addition, the sound instructions may be universally understandable by any person regardless of the language they use.

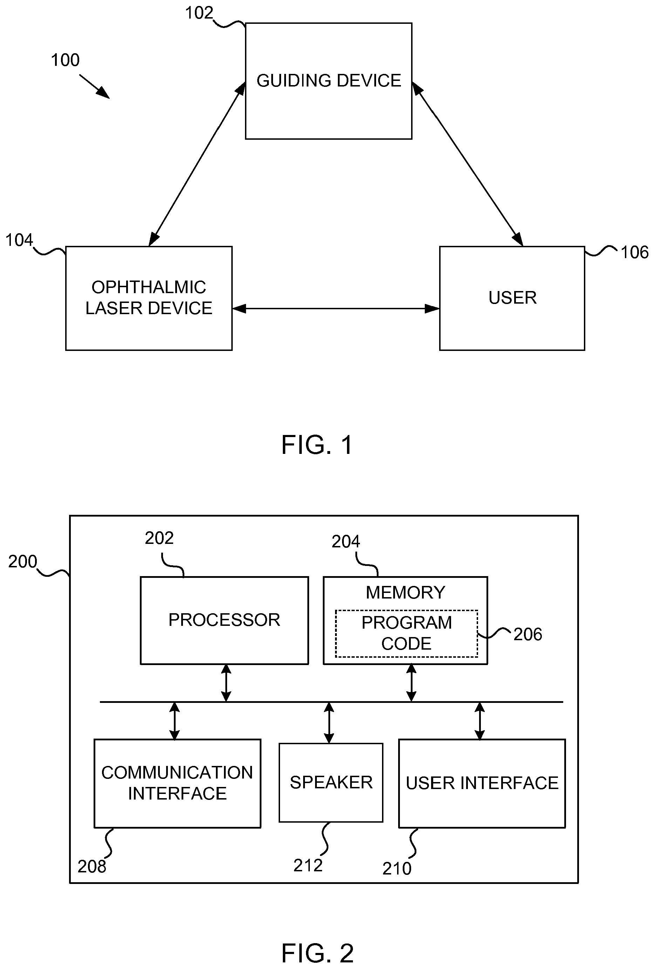

FIG. 1 illustrates an example embodiment of an ophthalmic laser therapy treatment system 100, according to an example embodiment. The ophthalmic laser therapy treatment system 100 may be configured for, for example, photothermal or photodynamic therapy treatment. The system 100 may be configured to enable management of a correct laser spot size for ophthalmic treatment based on voice guidance.

The laser spot size may refer to a diameter of the laser beam. The voice guidance may be based on non-speech sounds for an intuitive and quick adjustment procedure. The management may be based on sound signals provided by a guiding device 102 to a user 106 of an ophthalmic laser device 104. The voice guidance may be based on laser spot size parameters monitored by the guiding device 102. The user is an operator of the PDT device, such as a medical professional performing the treatment of the eye.

The system 100 comprises the ophthalmic laser device 104. The ophthalmic laser device 104 may be configured for laser activation of a photosensitive drug. In an embodiment, the ophthalmic laser device 104 may be a photodynamic therapy treatment device. The ophthalmic laser device 104 may comprise optic elements configured for laser delivery. In an embodiment, the ophthalmic laser device 104 may comprise a laser beam shaping unit coupled to a slit lamp. The laser beam shaping unit is a device configured to deliver a laser spot into the patient's eye through a contact lens. The slit lamp may comprise a laser source. The laser source is a high-intensity light source that can be focused into the eye. The laser beam shaping unit may comprise optical elements for shaping the laser beam. The laser beam shaping unit may further comprise at least one of mechanical control elements or an electrical circuitry arranged for controlling positions of the optical elements in relation to each other.

Shaping the laser beam may comprise changing the laser spot size. The laser beam shaping unit may comprise an adjustment ring or wheel configured to enable the user 106 to change a diameter of the laser spot size. The adjustment wheel may be coupled to the mechanical control arrangement configured to change position of the optical elements. The diameter of the laser spot size may be changed, for example, from 0.5 mm to 20 mm. The diameter may be changed steplessly or by predetermined steps in response to one or more user inputs.

The ophthalmic laser device 104 may be communicatively coupled with the guiding device 102, for example with a data connection. The guiding device 102 may be configured to obtain an indication of a current laser spot size value from the ophthalmic laser device 104. The current laser spot size may be received, for example, based on the diameter value selected from the adjustment wheel. The value of the laser spot size may be continuously monitored by the guiding device 102 based on sensor data.

The sensor data may be received, for example, from at least one electrical component configured to measure the spot size adjustment wheel position. The electrical component may be, for example, a potentiometer. In an embodiment, the sensor data may be obtained from two potentiometers for safety reasons. For example, one potentiometer could malfunction, and the failure may not be detected but when two potentiometers are used it may be checked that both give sensible values. The measurements may be converted to laser spot size by using a configuration table defined during calibration of the ophthalmic laser device. The configuration table may comprise multipliers for the measurement values. The calibration may be done by measuring the actual laser spot size diameter with a suitable spot size measurement tool and recording corresponding potentiometer values. In an embodiment, the guiding device 102 may be configured to receive the value of the laser spot size each time the position of the adjusting wheel is changed, e.g. in case the user 106 has rotated the wheel. Alternatively, the guiding device 102 may be configured to monitor the actual laser spot size on the retina of the patient. The actual laser spot size may be determined, for example, based on image data on the eye. In an embodiment, the slit lamp or the laser beam shaping unit may comprise a measurement device or a camera configured to provide measurement data associated with the size of the laser spot size.

The guiding device 102 may compare the obtained laser spot size value to a predetermined laser spot size required for treatment. The required laser spot size may be set by the user. In an embodiment, the guiding device 102 may be configured to obtain the required laser spot size based on manual user inputs. Alternatively, the required laser spot size may be obtained by the guiding device 102 wirelessly, for example, from a remote treatment management database storing parameters of the treatment session. The treatment session may refer to the time from preparation of the ophthalmic laser device 104 for active treatment to ending of the treatment. The treatment session may comprise activity time of the ophthalmic laser device, i.e. the time the ophthalmic laser device 104 is powered on. The required laser spot size may comprise a value range within which the laser spot size needs to remain. The range may be, for example, +/-0.5 mm of the required laser spot size.

When the guiding device 102 detects the current laser spot size and the required laser spot size do not match, the guiding device 102 may be configured to generate at least one sound signal. In an embodiment, the guiding device 102 may be configured to generate a sound distinguishable from the background noises, such as a loud or long beep as a warning indicating that the laser spot size is not correct.

In addition, the guiding device 102 may be configured to analyse the difference between the current and required laser spot size value. Further, the guiding device 102 may be configured to determine a sound signal or a sequence of sound signals indicative of the difference. The guiding device 102 may comprise an electrical circuitry configured to calculate the difference between the laser spot size values based on input data, determine characteristics of the sound signal based on the calculation, and output the sound signal as an electrical audio-frequency signal for production of audible voice instructions. In an embodiment, the guiding device 102 may comprise a unit configured to convert the audio-frequency signals into the equivalent audible sound waves. Alternatively, the guiding device 102 comprises a unit configured to transmit the audio-frequency signals to another device for the conversion.

Different types of sound signals may be determined by the guiding device 102 based on an amount of the difference. In an embodiment, characteristics of the sound signal are determined by the guiding device 102 based on the difference between the current and the required laser spot size. The difference may comprise a numerical difference between the laser spot size values as well as an indication of a direction of the difference, i.e. whether the current laser spot size is too large or too small compared to the required laser spot size. The guiding device 102 may be configured to generate the audio-frequency signals based on the determined characteristics.

In an embodiment, the guiding device 102 may detect a difference between the current and required laser spot size. Based on the detected difference, a warning sound is played by the guiding device 102. Alternatively, the guiding device 102 may be configured to send the sound signal for generation of the warning sound to another device, such as a speaker. The guiding device 102 may calculate that the current laser spot size is 2 mm too large. Based on the difference, the guiding device 102 may play, or cause the playing of, a repetitive high tone. Based on the high tone, the user 106 may know that the current laser spot size is too large and proceed to adjust the laser spot size by turning the adjusting wheel. When the adjusting wheel is turned, the guiding device 102 obtains an indication of the new current laser spot size. The guiding device 102 may calculate a new value difference between the required laser spot size and the new current laser spot size. The guiding device 102 may be configured to adjust the characteristics of the sound signal to indicate the new required adjustment. For example, the repetition pace and/or frequency of the tone may be lowered based on a lowered difference. Hence, the user 106 may know (s)he is changing the laser spot size value to the correct direction based on the sounds. The sounds may also indicate the required laser spot size is getting closer, for example, based on a change in the pace of the sounds.

FIG. 2 illustrates an example embodiment of a device 200 configured to practice one or more example embodiments. The device 200 may be, for example, the guiding device 102.

The device 200 may comprise at least one processor 202. The at least one processor may comprise, for example, one or more of various processing devices, such as for example a co-processor, a microprocessor, a controller, a digital signal processor (DSP), a processing circuitry with or without an accompanying DSP, or various other processing devices including integrated circuits such as, for example, an application specific integrated circuit (ASIC), a field programmable gate array (FPGA), a microcontroller unit (MCU), a hardware accelerator, a special-purpose computer chip, or the like.

The device 200 may further comprise at least one memory 204. The memory may be configured to store, for example, computer program code or the like, for example operating system software and application software. The memory may comprise one or more volatile memory devices, one or more non-volatile memory devices, and/or a combination thereof. For example, the memory may be embodied as magnetic storage devices (such as hard disk drives, magnetic tapes, etc.), optical magnetic storage devices, or semiconductor memories (such as mask ROM, PROM (programmable ROM), EPROM (erasable PROM), flash ROM, RAM (random access memory), etc.).

The device 200 may further comprise a communication interface 208 configured to enable the device 200 to transmit and/or receive information, to/from other devices. In an embodiment, the device 200 may be configured to receive parameter data from an ophthalmic laser device. The parameter data may be received, for example, based on a position of a laser spot size adjustment wheel of the ophthalmic laser device. The communication interface 208 may be configured to provide at least one wireless radio connection, such as for example a 3GPP mobile broadband connection (e.g. 3G, 4G, 5G). However, the communication interface 208 may be configured to provide one or more other types of connections, for example a wireless local area network (WLAN) connection such as for example standardized by IEEE 802.11 series or Wi-Fi alliance; a short range wireless network connection such as for example a Blue-tooth, NFC (near-field communication), or RFID connection; a wired connection such as for example a local area network (LAN) connection, a universal serial bus (USB) connection or an optical network connection, or the like; or a wired Internet connection. The communication interface 208 may comprise, or be configured to be coupled to, at least one antenna to transmit and/or receive radio frequency signals. One or more of the various types of connections may be also implemented as separate communication interfaces, which may be coupled or configured to be coupled to a plurality of antennas.

The device 200 may further comprise a user interface 210 comprising an input device and/or an output device. The input device may take various forms such a keyboard, a touch screen, or one or more embedded control buttons. The output device may comprise a speaker 212. The output device may further comprise, for example, a display, lights, or the like.

When the device 200 is configured to implement some functionality, some component and/or components of the device 200, such as for example the at least one processor and/or the memory, may be configured to implement this functionality. Furthermore, when the at least one processor 202 is configured to implement some functionality, this functionality may be implemented using program code 206 comprised, for example, in the memory 204.

The functionality described herein may be performed, at least in part, by one or more computer program product components such as software components. According to an embodiment, the device 200 comprises a processor or processor circuitry, such as for example a microcontroller, configured by the program code when executed to execute the embodiments of the operations and functionality described. Alternatively, or in addition, the functionality described herein can be performed, at least in part, by one or more hardware logic components. For example, and without limitation, illustrative types of hardware logic components that can be used include Field-programmable Gate Arrays (FPGAs), application-specific Integrated Circuits (ASICs), application-specific Standard Products (ASSPs), System-on-a-chip systems (SOCs), Complex Programmable Logic Devices (CPLDs), Graphics Processing Units (GPUs).

The device 200 may comprise means for performing at least one method described herein. In one example, the means comprises the at least one processor 202, the at least one memory 204 including program code 206 configured to, when executed by the at least one processor 202, cause the device 200 to perform the method.

The device 200 may comprise for example a computing device such as for example a server device, a client device, a mobile phone, a tablet computer, a laptop, or the like. Although the device 200 is illustrated as a single device it is appreciated that, wherever applicable, functions of the device 200 may be distributed to a plurality of devices.

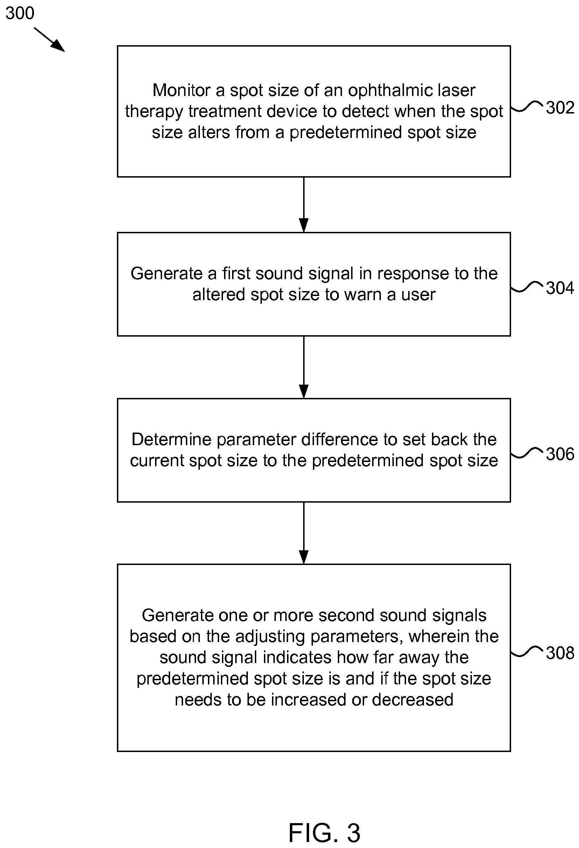

FIG. 3 illustrates an example of a method 300 for providing audible guidance by sound for a user of a device for ophthalmic laser therapy, according to an example embodiment. The method 300 may be executed, for example, by the device 200. The audible guidance may be performed by single sounds and sequences of the sounds determined by the device 200 based on input data. The sounds may comprise non-speech voices, such as beeps and musical sounds having different tones, lengths and/or sequences for indication of different information.

At 302, the method 300 comprises monitoring a laser spot size of the ophthalmic laser device. The method 300 may comprise obtaining a predetermined laser spot size as a basis for the monitoring function. The monitoring may comprise comparing the predetermined laser spot size to a current laser spot size to detect when the laser spot sizes fail to match.

When the laser spot size is altered during treatment, a first sound signal may be played as a warning at 304. The device 200 may determine treatment is ongoing when any input is received from the ophthalmic laser device. Characteristics of the warning sound may be selected such that it catches attention of the user of the ophthalmic laser device. In an embodiment, the first sound signal may be repeated frequently until a change of the current laser spot size is detected by the device 200. Alternatively, the warning sound may be played for a predetermined time before the process proceeds to 306.

At 306, the method 300 may comprise determining a parameter difference between the current laser spot size and the predetermined laser spot size. In an embodiment, the device 200 may be configured to calculate a numeral difference between the current and predetermined laser spot size. In addition, the device 200 may be configured to determine if the current laser spot size needs to be lowered or increased in order to reduce the difference and set back the predetermined laser spot size.

At 308, the method 300 may comprise generating one or more second sound signals based on the parameter difference. The second sound signal may be a non-speech sound. The second sound signal may indicate how far away the predetermined laser spot size or the amount of the required change is. When an adjustment wheel or similar arrangement is used for controlling the laser spot size, the sound signal may indicate if the laser spot size needs to be increased or decreased, i.e. to which direction the adjustment wheel needs to be turned.

In an embodiment, the method 300 comprises causing generation of sounds at a higher or lower frequency based on the difference between the current and predetermined laser spot size. In an embodiment, a higher tone of the second sound signal may indicate the current laser spot size needs to be increased. In an embodiment, a lower tone of the second sound signal may be configured to indicate the current laser spot size needs to be decreased.

In an embodiment, the method 300 may comprise determining a repetition frequency of the second sound signal. For example, if the required laser spot size is relatively far away, a plurality of second sound signals may be provided at a higher pace compared to a situation when the required laser spot size is closer to the current laser spot size. Alternatively, the repetition pace of the second sound signal may indicate if the current laser spot size is too high or too low. In an embodiment, the device 200 may determine an interval for the repetition of the second sound signals based on at least one of a magnitude of the parameter difference or required direction of the adjustment. In an embodiment, the number of provided second sound signals may indicate the number of stepwise parameter value adjustments required when turning the adjustment wheel to achieve the predetermined laser spot size. In an embodiment, the method may comprise determining a volume of the second sound signal based on the difference. For example, the sound may be first louder when the difference is large and start to become quieter when the difference decreases towards the required spot size value.

In an embodiment, the method 300 may comprise providing a third sound signal for confirmation when the required laser spot size is set. The user may then continue operating the ophthalmic laser device as usual. The device 200 may be configured to continue monitoring the laser spot size in case adjustment is again needed.

In an embodiment, the sound signals may comprise speech in addition to the non-speech sounds. In an embodiment, at least one of the warning sound or the confirmation sound may be provided as speech. For example, the device 200 may be configured to output: "Warning! Adjust the laser spot size". Thereafter, the adjustment guidance may be provided based on beep sounds, wherein the tone and/or output interval of the beeps indicate what kind of adjustment is required. When the user turns the adjusting wheel, the generated non-speech sounds may indicate if the laser spot size is changing to the correct direction and how close the laser spot size is to the required laser spot size. The adjustment may be performed quicker by the user compared to listening to a more time-consuming sentence instructions or by glancing at a display and/or the scale of the adjustment wheel. Further, safety may be increased due to automatic, noticeable and intuitive notification and guidance to set back the altered laser spot size. The user may only need to move a position of one hand to control the adjustment wheel during the treatment. If head movement was also required to check the situation, it could lead to movement of the other hand holding for example a contact lens.

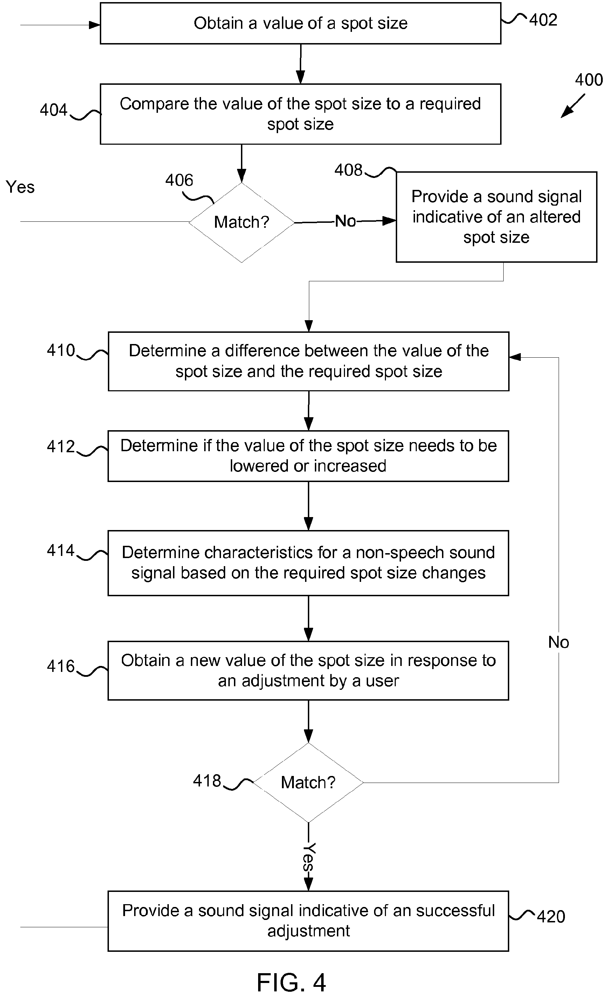

Figure 4 illustrates an example of a method 400 for determining sound instructions provided for a user of an ophthalmic laser therapy treatment device, according to an example embodiment. The method 400 may be executed, for example, by the device 200.

At 402, the method 400 comprises obtaining a value of a laser spot size. The laser spot size value may be received by the device 200 from the ophthalmic laser device, for example, based on a current position of an adjustment wheel for laser spot size selection. At least information associated with changes in the position of the adjustment wheel may be automatically obtained by the device 200. In an embodiment, the adjustment wheel may be coupled with a sensor arrangement providing sensor data for the device 200.

At 404, the method 400 may comprise comparing the value of the laser spot size to a required laser spot size. The required laser spot size may be stored by the device 200. The required laser spot size may be modified by the user, for example, based on treatment parameters.

At 406, the method 400 may comprise comparing the value of the laser spot size and the required laser spot size. At 408, the method may comprise providing a sound signal indicative of an altered laser spot size. The laser spot size is altered if it does not match the required laser spot size. Alternatively, the sound signal is provided when the laser spot size is not within a predetermined value range set for the laser spot size. The device 200 may be configured to create and dispatch the sound signal as an electrical signal based on the altered laser spot size. The electrical signal may be then converted by the device 200 or an external speaker device to generate an audible sound signal.

At 410, the method 400 may comprise determining a difference between the value of the laser spot size and the required laser spot size. At 412, the method may comprise determining if the value of the laser spot size needs to be lowered or increased.

Based on the calculations at 410 and 412, the method 400 may comprise determining characteristics for a non-speech sound signal. The characteristics may comprise a frequency, a repetition interval, a tone and/or an amplitude of the sound signal. The characteristics may be set to change based on the amount of the difference between the laser spot sizes and the direction of change to achieve the required laser spot size.

At 416, the method 400 may comprise obtaining a new value of the laser spot size in response to an adjustment by the user. The user may have turned the adjustment wheel to set the laser spot size according to the guidance provided by the non-speech signal (s) . When the adjustment wheel is turned, information about the new values of the current laser spot size is received by the device 200. While the values of the laser spot size differ from the required laser spot size, at 418, operations 410, 412, 414, 416 and 418 may be repeated.

When the required laser spot size value is successfully achieved, a sound signal indicative of the successful adjustment may be provided at 420. The device 200 may determine characteristics of the sound signal such that it is distinguishable from the non-speech sound signals at 414. The sound signals at 408 and 420 may be non-speech sounds. In addition, or alternatively, the sound signals at 408 and 420 may comprise speech.

Further features of the method(s) directly result for example from functionalities of the devices described throughout the specification and in the appended claims and are therefore not repeated here. Different variations of the method(s) may be also applied, as described in connection with the various example embodiments. It is obvious to a person skilled in the art that with the advancement of technology, the basic idea of the disclosure may be implemented in various ways. The disclosure and the embodiments are thus not limited to the examples described above, instead they may vary within the scope of the claims.

A device may be configured to perform or cause performance of any aspect of the method(s) described herein. Further, a computer program may comprise instructions for causing, when executed, a device to perform any aspect of the method(s) described herein. Further, a device may comprise means for performing any aspect of the method(s) described herein. According to an example embodiment, the means comprises at least one processor, and memory including program code, the at least one processor, and program code configured to, when executed by the at least one processor, cause performance of any aspect of the method(s).

Any range or device value given herein may be extended or altered without losing the effect sought. Also, any embodiment may be combined with another embodiment unless explicitly disallowed.

Although the subject matter has been described in language specific to structural features and/or acts, it is to be understood that the subject matter defined in the appended claims is not necessarily limited to the specific features or acts described above. Rather, the specific features and acts described above are disclosed as examples of implementing the claims and other equivalent features and acts are intended to be within the scope of the claims.

It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item may refer to one or more of those items.

The operations of the methods described herein may be carried out in any suitable order, or simultaneously where appropriate. Additionally, individual blocks may be deleted from any of the methods without departing from the scope of the subject matter described herein. Aspects of any of the embodiments described above may be combined with aspects of any of the other embodiments described to form further embodiments without losing the effect sought.

The term 'comprising' is used herein to mean including the method, blocks, or elements identified, but that such blocks or elements do not comprise an exclusive list and a method or apparatus may contain additional blocks or elements.

The terms 'automated', 'automatically', 'automatic' and variations thereof, as used herein, may refer to any process or operation done without human input when the process or operation is performed. However, a process or operation can be automatic, even though performance of the process or operation uses human input, if the input is received before performance of the process or operation.

Although subjects may be referred to as 'first' or 'second' subjects, this does not necessarily indicate any order or importance of the subjects. Instead, such attributes may be used solely for the purpose of making a difference between subjects.

It will be understood that the above description is given by way of example only and that various modifications may be made by those skilled in the art. The above specification, examples and data provide a complete description of the structure and use of exemplary embodiments. Although various embodiments have been described above with a certain degree of particularity, or with reference to one or more individual embodiments, those skilled in the art could make numerous alterations to the disclosed embodiments without departing from scope of this specification.

## Claims

1. A device (200), the device comprising at least one processor (202) and at least one memory (204), the memory comprising program code (206) configured to, when executed by the processor (202), cause the device (200) at least to:
monitor a current laser spot size of an ophthalmic laser therapy treatment device based on sensor data;
cause output of a first sound signal in response to detecting the current laser spot size differs from a required laser spot size;
determine a required parameter change to set the value of the current laser spot size to the required laser spot size;
**characterized in that** the device (200) is further caused to:
determine characteristics of a second sound signal based on the required parameter change, wherein the second sound signal comprises a non-speech sound; and
cause output of the second sound signal, wherein characteristics of the second sound signal are indicative of a magnitude of the required parameter change and whether the laser spot size needs to be increased or decreased.

2. The device of claim 1, wherein the characteristics of the second sound signal comprise a higher or a lower frequency depending on whether the laser spot size needs to be increased or decreased.

3. The device of claim 1 or 2, wherein the characteristics of the second sound signal comprise a different repetition interval based on the magnitude of the required parameter change.

4. The device of any of claims 1 to 3, wherein the characteristics of the second sound signal comprises a different volume level based on the magnitude of the required parameter change.

5. The device of claim 1, wherein the characteristics of the second sound signal comprise setting at least two of a different frequency, a different repetition interval or a different volume level based on different magnitudes of the required parameter changes and whether the laser spot size needs to be increased or decreased.

6. The device of any of claims 1 to 5, wherein the at least one memory (204) and the program code (206) are further configured to, when executed by the processor (202), cause the device (200) to:
detect when the current laser spot size corresponds to the predetermined laser spot size; and
cause output of a third sound signal indicative of a successful adjustment.

7. The device of claim 6, wherein at least one of the first sound signal or the third sound signal comprises at least one of a speech or a non-speech sound.

8. The device of any of claims 1 to 7, wherein the at least one memory (204) and the program code (206) are further configured to, when executed by the processor (202), cause the device (200) to:
detect the current laser spot size is changed in response to an adjustment of the laser spot size made by a user;
determine a new required parameter change; and
change the characteristics of the second sound signal based on the new required parameter change.

9. The device of any of claims 1 to 8, wherein monitoring the current laser spot size comprises obtaining a value of the current laser spot size based on a position of a laser spot size adjustment wheel of the ophthalmic laser therapy treatment device and comparing the value to the required laser spot size value.

10. A method carried out by a device (200) for monitoring laser spot size of an ophthalmic laser therapy treatment device, the method comprising:
monitoring a current laser spot size of the ophthalmic laser therapy treatment device based on sensor data;
causing output of a first sound signal in response to detecting the current laser spot size differs from a required laser spot size;
determining a required parameter change to set the value of the current laser spot size to the required laser spot size; **characterized in that** the method further comprises:
determining characteristics of a second sound signal based on the required parameter change, wherein the second sound signal comprises a non-speech sound; and
causing output of the second sound signal, wherein characteristics of the second sound signal are indicative of a magnitude of the required parameter change and whether the laser spot size needs to be increased or decreased.

11. The method of claim 10, wherein determining the characteristics of the second sound signal comprises at least one of:
a higher or a lower frequency depending on whether the laser spot size needs to be increased or decreased;
a different repetition interval based on the magnitude of the required parameter change;
a different volume level based on the magnitude of the required parameter change; or
at least two of a different frequency, a different repetition interval, or a different volume level based on different magnitudes of the required parameter changes and whether the laser spot size needs to be increased or decreased.

12. The method of claim 10 or 11, further comprising:
detecting when the current laser spot size corresponds to the predetermined laser spot size; and
causing output of a third sound signal indicative of a successful adjustment.

13. The method of any of claims 10 to 12, comprising:
detecting the current laser spot size is changed in response to an adjustment of the laser spot size made by a user; and
changing the characteristics of the second sound signal based on a new required parameter change.

14. The method of any of claims 10 to 13, wherein monitoring the current laser spot size comprises obtaining a value of the current laser spot size based on a position of a laser spot size adjustment wheel of the ophthalmic laser therapy treatment device and comparing the value to the predetermined laser spot size value.

15. A system (100), comprising the device (200) according to claim 1 communicatively coupled to an ophthalmic laser therapy device.

## Patentansprüche

1. Vorrichtung (200), wobei die Vorrichtung mindestens einen Prozessor (202) und mindestens einen Speicher (204) umfasst, wobei der Speicher Programmcode (206) umfasst, der so konfiguriert ist, dass er bei Ausführung durch den Prozessor (202) die Vorrichtung (200) dazu veranlasst, mindestens zum:
Überwachen der aktuellen Laserspotgröße einer ophthalmologischen Lasertherapievorrichtung anhand von Sensordaten;
Veranlassen ein erstes Tonsignal auszugeben, wenn bestimmt wird, dass die aktuelle Laserspotgröße von der erforderlichen Laserspotgröße abweicht;
Bestimmen einer erforderlichen Parameteränderung , um den Wert der aktuellen Laserspotgröße auf die gewünschte Laserspotgröße einzustellen;
**dadurch gekennzeichnet, dass** die Vorrichtung (200) weiter dazu veranlasst wird:
Bestimmen der Eigenschaften eines zweiten Tonsignals auf der Grundlage der erforderlichen Parameteränderung, wobei das zweite Tonsignal ein Nicht-Sprachgeräusch umfasst; und
Veranlassen das zweite Tonsignal auszugeben, wobei die Eigenschaften des zweiten Tonsignals Aufschluss über das Ausmaß der erforderlichen Parameteränderung geben und darüber, ob die Laserspotgröße vergrößert oder verkleinert werden muss.

2. Vorrichtung nach Anspruch 1, wobei die Eigenschaften des zweiten Tonsignals eine höhere oder eine niedrigere Frequenz aufweisen, je nachdem, ob die Laserspotgröße vergrößert oder verkleinert werden soll.

3. Vorrichtung nach Anspruch 1 oder 2, wobei die Eigenschaften des zweiten Tonsignals ein unterschiedliches Wiederholungsintervall umfassen, das sich nach dem Ausmaß der erforderlichen Parameteränderung richtet.

4. Vorrichtung nach einem der Ansprüche 1 bis 3, wobei die Eigenschaften des zweiten Tonsignals einen unterschiedlichen Lautstärkepegel umfassen, der sich nach dem Ausmaß der erforderlichen Parameteränderung richtet.

5. Vorrichtung nach Anspruch 1, wobei die Merkmale des zweiten Tonsignals die Einstellung von mindestens zwei der folgenden Parameter umfassen: eine unterschiedliche Frequenz, ein unterschiedliches Wiederholungsintervall oder eine unterschiedliche Lautstärke, basierend auf unterschiedlichen Größenordnungen der erforderlichen Parameteränderungen und darauf, ob die Laserspotgröße vergrößert oder verkleinert werden muss.

6. Vorrichtung nach einem der Ansprüche 1 bis 5, wobei der mindestens eine Speicher (204) und der Programmcode (206) weiter so konfiguriert sind, dass sie bei Ausführung durch den Prozessor (202) der Vorrichtung (200) veranlassen zum:
Erkennen, wann die aktuelle Laserspotgröße der vorgegebenen Laserspotgröße entspricht; und
Veranlassen, ein drittes Tonsignal auszugeben, das eine erfolgreiche Einstellung anzeigt.

7. Vorrichtung nach Anspruch 6, wobei mindestens eines der ersten oder dritten Tonsignale mindestens einen Sprach- oder Nicht-Sprachton umfasst.

8. Vorrichtung nach einem der Ansprüche 1 bis 7, wobei der mindestens eine Speicher (204) und der Programmcode (206) weiter so konfiguriert sind, dass sie bei Ausführung durch den Prozessor (202) der Vorrichtung (200) veranlassen zum:
Erkennen, dass sich die aktuelle Laserspotgröße als Reaktion auf eine vom Benutzer vorgenommene Anpassung der Laserspotgröße ändert;
Bestimmen einer neuen erforderlichen Parameteränderung; und
Ändern der Eigenschaften des zweiten Tonsignals entsprechend der neuen erforderlichen Parameteränderung.

9. Vorrichtung nach einem der Ansprüche 1 bis 8, wobei das Überwachen der aktuellen Laserspotgröße das Ermitteln eines Wertes für die aktuelle Laserspotgröße auf der Grundlage der Position eines Einstellrads für die Laserspotgröße an der ophthalmologischen Lasertherapie-Behandlungsvorrichtung sowie den Vergleich dieses Wertes mit dem Sollwert für die Laserspotgröße umfasst.

10. Von einer Vorrichtung (200) ausgeführtes Verfahren zur Überwachung der Laserspotgröße eines Geräts zur ophthalmologischen Lasertherapie, wobei das Verfahren umfasst:
Überwachen der aktuellen Laserspotgröße des Geräts zur ophthalmologischen Lasertherapie auf der Grundlage von Sensordaten;
Veranlassen, ein erstes Tonsignal auszugeben, wenn bestimmt wird, dass die aktuelle Laserspiegelgröße von der erforderlichen Laserspiegelgröße abweicht;
Bestimmen einer erforderlichen Parameteränderung, um den Wert der aktuellen Laserspotgröße auf die gewünschte Laserspotgröße einzustellen;
**dadurch gekennzeichnet, dass** das Verfahren weiter umfasst:
Bestimmen von Merkmalen eines zweiten Tonsignals auf der Grundlage der erforderlichen Parameteränderung, wobei das zweite Tonsignal einen Nicht-Sprachton umfasst; und
Veranlassen, ein zweites Tonsignal auszugeben, wobei die Eigenschaften des zweiten Tonsignals Aufschluss über das Ausmaß der erforderlichen Parameteränderung geben und darüber, ob die Laserspotgröße vergrößert oder verkleinert werden muss.

11. Verfahren nach Anspruch 10, wobei das Bestimmen der Eigenschaften des zweiten Tonsignals mindestens einen der folgenden Schritte umfasst:
eine höhere oder niedrigere Frequenz, je nachdem, ob die Laserpunktgröße vergrößert oder verkleinert werden soll;
ein anderes Wiederholungsintervall, basierend auf dem Ausmaß der erforderlichen Parameteränderung;
eine unterschiedliche Lautstärke, basierend auf dem Ausmaß der erforderlichen Parameteränderung; oder
mindestens zwei von einer unterschiedlichen Frequenz, einem unterschiedlichen Wiederholungsintervall oder einer unterschiedlichen Lautstärke, je nach dem Ausmaß der erforderlichen Parameteränderungen und je nachdem, ob die Laserspotgröße vergrößert oder verkleinert werden muss.

12. Verfahren nach Anspruch 10 oder 11, weiter umfassend:
Bestimmen, wann die aktuelle Laserspiegelgröße der vorgegebenen Laserspiegelgröße entspricht; und
Veranlassen, ein drittes Tonsignal auszugeben, das eine erfolgreiche Einstellung anzeigt.

13. Verfahren nach einem der Ansprüche 10 bis 12, umfassend:
Bestimmen der aktuellen Laserspotgröße, die sich als Reaktion auf eine vom Benutzer vorgenommene Anpassung der Laserspotgröße ändert; und
Ändern der Eigenschaften des zweiten Tonsignals basierend auf einer neuen erforderlichen Parameteränderung.

14. Verfahren nach einem der Ansprüche 10 bis 13, wobei das Überwachen der aktuellen Laserspotgröße das Ermitteln eines Wertes für die aktuelle Laserspotgröße auf der Grundlage einer Position eines Einstellrads für die Laserspotgröße an der ophthalmologischen Lasertherapie-Behandlungsvorrichtung und das Vergleichen dieses Wertes mit dem vorgegebenen Wert für die Laserspotgröße umfasst.

15. System (100), umfassend die Vorrichtung (200) nach Anspruch 1 , die kommunikativ mit einer ophthalmologischen Lasertherapie-Vorrichtung verbunden ist.

## Revendications

1. Dispositif (200), le dispositif comprenant au moins un processeur (202) et au moins une mémoire (204), la mémoire comprenant un code de programme (206) configuré pour, lorsqu'il est mis en œuvre par le processeur (202), amener le dispositif (200) au moins à :
surveiller une taille actuelle du spot laser d'un dispositif de traitement thérapeutique laser ophtalmique sur la base de données de capteur ;
provoquer l'émission d'un premier signal sonore en réponse à la détection selon laquelle la taille actuelle du spot laser diffère d'une taille de spot laser requise ;
déterminer une modification de paramètre requise pour régler la valeur de la taille actuelle du spot laser sur la taille de spot laser requise ;
**caractérisé en ce que** le dispositif (200) est en outre amené à :
déterminer des caractéristiques d'un second signal sonore sur la base de la modification de paramètre requise, dans lequel le second signal sonore comprend un son non vocal ; et
provoquer l'émission du second signal sonore, dans lequel des caractéristiques du second signal sonore sont représentatives de l'ampleur de la modification de paramètre requise et du fait que la taille du spot laser doit être augmentée ou diminuée.

2. Dispositif selon la revendication 1, dans lequel les caractéristiques du second signal sonore comprennent une fréquence plus élevée ou plus basse selon que la taille du spot laser doit être augmentée ou diminuée.

3. Dispositif selon la revendication 1 ou la revendication 2, dans lequel les caractéristiques du second signal sonore comprennent un intervalle de répétition différent sur la base de l'ampleur de la modification de paramètre requise.

4. Dispositif selon l'une quelconque des revendications 1 à 3, dans lequel les caractéristiques du second signal sonore comprennent un niveau de volume différent sur la base de l'ampleur de la modification de paramètre requise.

5. Dispositif selon la revendication 1, dans lequel les caractéristiques du second signal sonore comprennent le réglage d'au moins deux parmi une fréquence différente, un intervalle de répétition différent ou un niveau de volume différent sur la base de différentes ampleurs des modifications de paramètre requises et selon que la taille du spot laser doit être augmentée ou diminuée.

6. Dispositif selon l'une quelconque des revendications 1 à 5, dans lequel au moins une mémoire (204) et le code programme (206) sont en outre configurés pour, lorsqu'ils sont exécutés par le processeur (202), amener le dispositif (200) à :
détecter lorsque la taille actuelle du spot laser correspond à la taille de spot laser prédéterminée ; et
provoquer l'émission d'un troisième signal sonore représentatif d'un réglage réussi.

7. Dispositif selon la revendication 6, dans lequel au moins l'un parmi le premier signal sonore ou le troisième signal sonore comprend au moins l'un parmi un son vocal ou un son non vocal.

8. Dispositif selon l'une quelconque des revendications 1 à 7, dans lequel au moins une mémoire (204) et le code programme (206) sont en outre configurés pour, lorsqu'ils sont exécutés par le processeur (202), amener le dispositif (200) à :
détecter que la taille actuelle du spot laser est modifiée en réponse à un réglage de la taille du spot laser effectué par un utilisateur ;
déterminer une nouvelle modification de paramètre requise ; et
modifier les caractéristiques du second signal sonore sur la base de la nouvelle modification de paramètre requise.

9. Dispositif selon l'une quelconque des revendications 1 à 8, dans lequel la surveillance de la taille actuelle du spot laser comprend l'obtention d'une valeur de la taille actuelle du spot laser sur la base d'une position d'une molette de réglage de la taille du spot laser du dispositif de traitement thérapeutique laser ophtalmique et la comparaison de la valeur avec la valeur requise de la taille du spot laser.

10. Procédé mis en œuvre par un dispositif (200) pour surveiller la taille d'un spot laser d'un dispositif de traitement thérapeutique laser ophtalmique, le procédé comprenant :
la surveillance d'une taille actuelle de spot laser du dispositif de traitement thérapeutique laser ophtalmique sur la base de données de capteur ;
la provocation de l'émission d'un premier signal sonore en réponse à la détection selon laquelle la taille actuelle du spot laser diffère d'une taille de spot laser requise ;
la détermination d'une modification de paramètre requise pour régler la valeur de la taille actuelle du spot laser sur la taille de spot laser requise ;
**caractérisé en ce que** le procédé comprend en outre :
la détermination de caractéristiques d'un second signal sonore sur la base de la modification de paramètre requise, dans lequel le second signal sonore comprend un son non vocal ; et
la provocation de l'émission du second signal sonore, dans lequel les caractéristiques du second signal sonore sont représentatives de l'ampleur de la modification de paramètre requise et du fait que la taille du spot laser doit être augmentée ou diminuée.

11. Procédé selon la revendication 10, dans lequel la détermination des caractéristiques du second signal sonore comprend au moins l'un parmi :
une fréquence plus élevée ou plus basse selon que la taille du spot laser doit être augmentée ou diminuée ;
un intervalle de répétition différent sur la base de l'ampleur de la modification de paramètre requise ;
un niveau de volume différent sur la base de l'ampleur de la modification de paramètre requise ; ou
au moins deux parmi une fréquence différente, un intervalle de répétition différent ou un niveau de volume différent sur la base de différentes ampleurs des modifications de paramètre requises et selon que la taille du spot laser doit être augmentée ou diminuée.

12. Procédé selon la revendication 10 ou la revendication 11, comprenant en outre :
la détection du moment où la taille actuelle du spot laser correspond à la taille de spot laser prédéterminée ; et
la provocation de l'émission d'un troisième signal sonore représentatif d'un réglage réussi.

13. Procédé selon l'une quelconque des revendications 10 à 12, comprenant :
la détection du fait que la taille actuelle du spot laser est modifiée en réponse à un réglage de la taille du spot laser effectué par un utilisateur ; et
la modification des caractéristiques du second signal sonore sur la base d'une nouvelle modification de paramètre requise.

14. Procédé selon l'une quelconque des revendications 10 à 13, dans lequel la surveillance de la taille actuelle du spot laser comprend l'obtention d'une valeur de la taille actuelle du spot laser sur la base d'une position d'une molette de réglage de la taille du spot laser du dispositif de traitement thérapeutique laser ophtalmique et la comparaison de la valeur avec la valeur prédéterminée de la taille du spot laser.

15. Système (100), comprenant le dispositif (200) selon la revendication 1, couplé de manière communicante à un dispositif de traitement thérapeutique laser ophtalmique.
